(19) Europäisches Patentamt
European Patent Office
Office européen des brevets

(11) Veröffentlichungsnummer: **0 279 893 B1**

(12) **EUROPÄISCHE PATENTSCHRIFT**

(45) Veröffentlichungstag der Patentschrift: **18.12.91**

(51) Int. Cl.5: **F23Q 2/32**, A61F 6/02

(21) Anmeldenummer: **87109257.3**

(22) Anmeldetag: **26.06.87**

Verbunden mit 87905727.1/0303626
(europäische
Anmeldenummer/Veröffentlichungsnummer)
durch Entscheidung vom 06.07.89.

(54) **Feuerzeug mit Kondom.**

(30) Priorität: **27.02.87 DE 8703096 U**

(43) Veröffentlichungstag der Anmeldung:
**31.08.88 Patentblatt 88/35**

(45) Bekanntmachung des Hinweises auf die
Patenterteilung:
**18.12.91 Patentblatt 91/51**

(84) Benannte Vertragsstaaten:
**AT BE CH DE ES FR GB GR IT LI LU NL SE**

(56) Entgegenhaltungen:
**CH-A- 662 724**
**CH-A- 662 875**
**DE-C- 441 927**
**US-A- 4 305 118**
**US-A- 4 583 939**

(73) Patentinhaber: **Söffner, Georg**
**Loristrasse 3**
**W-8000 München 2(DE)**

(72) Erfinder: **Söffner, Georg**
**Loristrasse 3**
**W-8000 München 2(DE)**

(74) Vertreter: **Diehl, Hermann Dr. et al**
**Diehl & Glaeser, Hiltl & Partner Flüggenstrasse 13**
**W-8000 München 19(DE)**

## Beschreibung

Die Erfindung betrifft ein Wegwerffeuerzeug mit Kondom.

Um einer weiteren Ausbreitung von Infektionskrankheiten vorzubeugen, die vornehmlich durch Geschlechtsverkehr übertragen werden, wie beispielsweise dem sog. AIDS-Virus, wird die Verwendung von Kondomen seitens der staatlichen Gesundheitsbehörden und der Ärzteschaft in zunehmend verstärktem Maße empfohlen, da nach Meinung der Fachleute nur auf diese Weise einer weiteren epidemieartigen Ausbreitung des Virus entgegengewirkt werden kann.

Da die Zufälligkeiten des Lebens es nicht voraussehbar machen, wann jeweils das Kondom benötigt wird, empfiehlt es sich, sicherheitshalber jeweils ein solches mit sich zu führen. Obwohl in den letzten Jahren eine zunehmende Liberalisierung, auch bezüglich des Sexualbereichs, in der Bevölkerung stattgefunden hat, gehen die Auffassungen dahingehend doch weit auseinander, was gesellschaftlich gut und schicklich ist. Es wird daher zumindest zum gegenwärtigen Zeitpunkt von einem Großteil der Bevölkerung noch als peinlich empfunden, wenn bei der Entnahme eines Gegenstands, wie eines Schlüssels, einer Geldbörse oder eines Ausweises aus einer Tasche versehentlich ein auch durch den Aufdruck als solches gekennzeichnetes Päckchen von Kondomen sichtbar wird oder herausfällt.

Ein weiterer Nachteil besteht darin, daß die gegenwärtigen Verpackungen zwar einen gewissen Schutz davor bieten, daß das Kondom während des Transports in einer Tasche oder einem ähnlichen Behältnis beschädigt wird, was jedoch bei längerem Nichtgebrauch nicht ganz auszuschließen ist.

Der vorliegenden Erfindung liegt daher die Aufgabe zugrunde, ein Behältnis für Kondome zu schaffen, das zum einen die Kondome gegen Beschädigungen schützt und das andererseits vom Herrn und der Dame unauffällig und unaufdringlich mitführbar ist und Dritten gegenüber nicht ohne weiteres erkennen läßt, daß der Träger in dem Behältnis ein Kondom mit sich führt. Zu diesem Zwecke wurde bereits im Jahre 1926 in der DE-C-441 927 der Vorschlag für einen Taschenbehälter mit Deckel zum unauffälligen Verwahren hygienischer Hilfsmittel, wie Gummischutzmittel oder dgl., gemacht.

Der Taschenbehälter enthält einen Einsatz, der zum Einlegen eines beim Öffnen des Deckels sichtbaren und einen anderen Zweck des Behälters vortäuschenden Gegenstandes eingerichtet ist und der zwischen seinem und dem Behälterboden so viel Zwischenraum frei läßt, als zur Unterbringung der zu verbergenden Schutzmittel nötig ist. Bei dem bekannten Taschenbehälter ist es nicht ersichtlich, welch anderer kleiner Gegenstand zweckmäßigerweise und unverfänglich in dem beim Öffnen des Deckels sichtbaren Scheinbehälter aufgenommen werden sollte, und jeder kleine darin aufgenommene Gegenstand würde Gefahr laufen, bei der Entnahme des Gummischutzmittels herauszufallen.

Aus der US-A-4,583,939 ist es ferner bekannt, in einem Feuerzeug einen Parfumzerstäuber unterzubringen.

Die vorliegende Erfindung löst die vorstehend erwähnte Aufgabe durch ein Wegwerffeuerzeug mit Kondom gemäß den unabhängigen Patentansprüchen 1 und 5. Bevorzugte Weiterbildungen der Erfindung sind den abhängigen Patentansprüchen zu entnehmen.

Gemäß einer ersten besonders bevorzugten Ausführungsform der Erfindung enthält das Feuerzeug angrenzend an den Brennstofftank eine mit einer wiederverschließbaren Entnahmeöffnung versehene Kammer, die das eng zusammengewickelte Kondom und dessen mit einer Entnahmelasche versehene Schutzhülle aufnimmt.

Als besonders günstig hat es sich erwiesen, wenn an der Unterseite des Feuerzeugs eine Klappe angebracht ist, welche zumindest einen Teil des Bodens des Feuerzeugs bildet. Die Klappe weist zweckmäßigerweise eine lösbare Arretierung auf.

Gemäß einer weiteren Ausgestaltung ist die im Feuerzeug angebrachte Kammer an der Seitenfläche und/oder der Unterseite des Feuerzeugs offen, wobei diese Öffnung durch eine über dem Körper des Feuerzeugs aufschiebbare Hülse abdeckbar ist. Das Aufschieben der Hülse auf den in der Regel zylindrischen Grundkörper des Feuerzeugs erfolgt in der Regel von unten. Bei geeigneter Ausgestaltung kann dies jedoch auch von oben her geschehen oder die Hülse ist nach Art einer federnden Klammer ausgebildet, die seitlich auf den Grundkörper des Feuerzeugs aufsteckbar ist.

Gemäß einer zweiten besonders vorteilhaften Ausführungsform ist die Kammer zur Aufnahme des Kondoms hermetisch abgeschlossen, wobei ein Zugang zum Kammerinneren lediglich durch Zerstörung der Kammerwandung längs zumindest einer Sollbruchstelle möglich ist, wodurch die Sterilität des Kondoms sichergestellt bleibt. Um die Entnahme zu erleichtern, ist an der Außenseite des Feuerzeugs eine Lasche zum Aufreißen der Kammerwandung längs der genannten Sollbruchstelle vorgesehen. Der Zugang zur Kammer ist dabei mit Vorzug im Seitenbereich des Feuerzeugs vorgesehen.

Bei Gasfeuerzeugen erstreckt sich die Kammer vorzugsweise bis unter die Federkammer für die Ventilrückstellfeder, wobei die Federkammer, welche den Feuerstein nach oben drückt, im Bereich der Wandung der Kammer angebracht ist, vorzugsweise in demjenigen Wandungsbereich, welcher die Kammer für die Aufnahme des Kondoms von der Brennstoffkammer trennt.

Das Feuerzeug kann auch dergestalt ausgebildet sein, daß die Kammer zur Aufnahme des Kondoms den gesamten unteren Bereich des Feuerzeugs ausfüllt. Für Nichtraucher genügt es, das Feuerzeug derart auszubilden, daß der Brennstofftank nur einen geringen Teil des Grundkörpers ausmacht, so daß im Vergleich zu normalen, von Rauchern benutzten Feuerzeugen nur eine begrenzte Zahl von Zündvorgängen möglich ist, was ausreicht, um einem Gast gelegentlich Feuer zu geben, wenn der Benutzer selbst nicht raucht.

Als günstig hat es sich erwiesen, wenn der Brennstofftank und die Wandung der Kammer aus Kunststoff bestehen, wobei sie vorzugsweise einstückig ausgebildet sind und dieses einstückige Kunststoffteil die restlichen mechanischen Bauteile des Feuerzeugs haltert.

Zweckmäßigerweise ist zumindest die Außenwand des Kunststoffteils undurchsichtig, so daß das Kondom nicht ohne weiteres sichtbar ist. Gemäß einer alternativen Ausgestaltung wird die Außenwand zumindest der zur Aufnahme des Kondoms vorgesehenen Kammer aus einem durchsichtigen Kunststoff gefertigt, wobei insbesondere bei einer Aufnahme des Kondoms in einer farbigen oder gemusterten Schutzhülle ein besonderer optischer Effekt des Feuerzeugs erzielt wird, ohne daß Dritte direkt erkennen können, daß das Feuerzeug auch ein Kondom enthält und wobei auch umgekehrt für den Benutzer angezeigt ist, wenn das entnommene Kondom durch ein neues ersetzt werden soll.

Weitere Einzelheiten und Ausgestaltungen der Erfindung ergeben sich aus den beiliegenden Zeichnungen. Darin zeigen:

Fig. 1 ein Wegwerffeuerzeug mit Kondom in teilgeschnittener Längsansicht, wobei in Fig. 1a eine wiederverschließbare Öffnung an der Unterseite des Feuerzeugs und in Fig. 1b eine aufreißbare Öffnung im Seitenbereich des Feuerzeugs vorgesehen ist.

Fig. 2 im Längsschnitt eine weitere Ausführungsform eines Feuerzeugs mit Kondomen, bei welcher eine Kammer zur Aufnahme mehrerer Kondome im unteren Bereich des Feuerzeugs angebracht ist.

Fig. 3 im Längsschnitt den unteren Bereich eines weiteren Ausführungsbeispiels für ein Feuerzeug mit Kondom.

In den Zeichnungen sind gleiche Teile jeweils mit gleichen Bezugzeichen versehen.

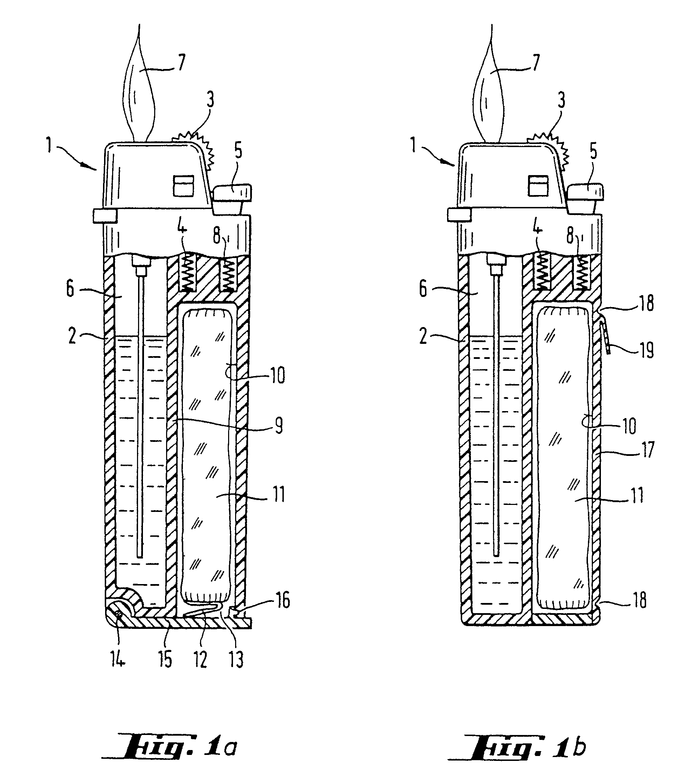

In Fig. 1a ist ein Feuerzeug 1 gezeigt, das aus einem Kunststoffbehälter 2 besteht, auf dessen Oberseite die mechanischen Teile des Feuerzeugs angebracht sind. Diese enthalten in der Regel eine drehbar gelagerte Rändelschraube 3, die mit einem Reibungsbereich mit einem nicht gezeigten Feuerstein in Verbindung tritt, der mittels einer Feder 4 in Richtung auf den Reibungsbereich 3 gedrückt wird. Durch einen Betätigungshebel 5 wird ein nicht näher gezeigtes Ventil geöffnet, welches einen Brennstoff in nicht näher dargestellter Weise aus einem Brennstofftank 6 ausströmen läßt, so daß der erzeugte Funke den gasförmigen Brennstoff erhitzt und zur Erzeugung der Flamme 7 führt. Eine Feder 8 dient zur Rückführung der Betätigungstaste 5 in die Schließstellung, so daß nach erfolgtem Zündvorgang die Flamme 7 automatisch gelöscht wird. Der Behälter 2, in dessen oberem Bereich die Federn 4 und 8 in entsprechenden Federkäfigen geführt sind, enthält eine durch eine Längswandung 9 abgetrennte Kammer 10 zur Aufnahme eines Kondoms 11, das von einer Schutzhülle umschlossen ist, die an ihrem unteren Ende eine Lasche 12 enthält, die ein Herausziehen des Kondoms aus der Kammer 10 durch eine am unteren Ende des Feuerzeugs angebrachte Austrittsöffnung 13 ermöglicht, welche durch einen an einem Zapfen 14 drehbar angelenkten Deckel 15 wiederverschließbar ist, wobei ein Nocken-Nut-Mechanismus 16 eine Arretierung des Deckels 15 bildet.

Bei dem in Fig. 1b gezeigten Feuerzeug ist die Kammer 10 hermetisch abgeschlossen. An der nach außen gekehrten Seitenwandung der Kammer 10 ist die Wandung des Behälters mit einer umlaufenden Sollbruchstelle 18 versehen, wobei der von der Sollbruchstelle 18 umgrenzte Bereich 17 eine zusätzliche Lasche 19 trägt, mittels derer der Wandungsbereich 17 aus der Behältniswand 2 herausreißbar ist. Das Kondom 11 ist steril in der Kammer 10 eingeschlossen, bis das Wandungsteil 17 herausgerissen wird. Diese Ausgestaltung ermöglicht kein Wiederauffüllen der Kammer 10 mit einem weiteren Kondom, was nach der Ausführungsform nach Fig. 1a möglich ist.

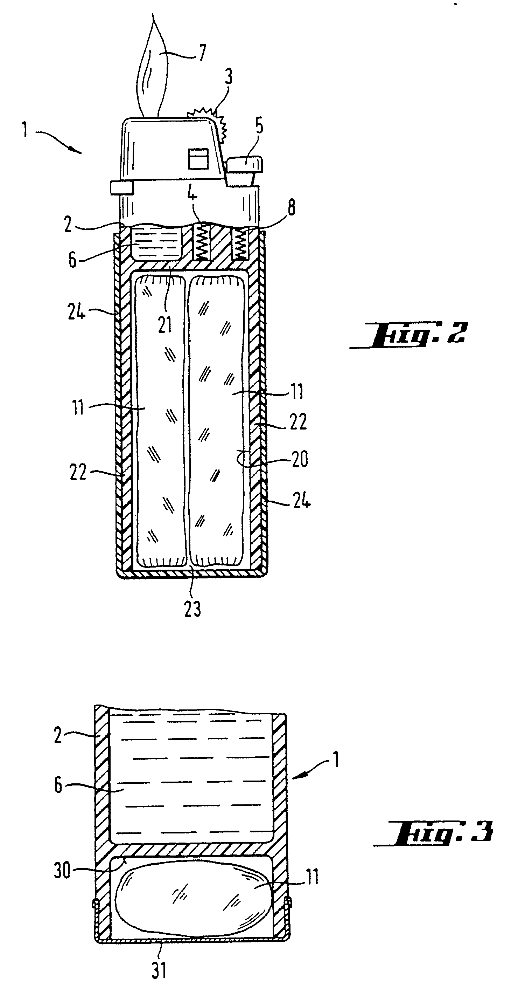

Bei dem in Fig. 2 gezeigten Feuerzeug 21 ist der Brennstofftank 6 gegenüber den in den Fig. 1a und 1b gezeigten Ausführungsformen stark verkleinert zugunsten einer Kammer 20, die den gesamten unteren Bereich des Feuerzeugs ausfüllt und im dargestellten Falle zwei Kondome 11 aufnimmt. Die Kammer 20 endet mit ihrer oberen Wandung 21 unmittelbar unter den Gehäusen für die Federn 4 und 8 und dem Boden des Brennstofftanks 6. Sie wird seitlich durch die zylindrische Außenwandung 22 des Feuerzeugkörpers gebildet, die am unteren Ende eine durchgehende Öffnung 23 freiläßt. Die Öffnung 23 wird durch Aufschieben einer Hülse 24 verschlossen, deren zylindrische Außenwandung der zylindrischen Außenkontur des Feuerzeugkörpers entspricht und deren Unterseite die Öffnung 23 abdeckt. Durch Herausziehen des Feuerzeugkörpers aus der Hülse 24 wird die Öffnung 23 freigelegt und die Kondome 11 können der Kammer 20 entnommen werden. In gleicher Weise ist ein Nachfüllen von Kondomen in die Kammer 20 möglich. Die in Fig. 2 dargestellte Ausführungsform ist bevorzugt für Nichtraucher gedacht, die gelegentlich mit ihrem Feuerzeug Dritten Feuer geben können, bei dem jedoch die Hauptfunktion die des Behältnisses zur Aufnahme mehrerer Kondome ist.

Bei der in Fig. 3 dargestellten Ausführungsform ist am unteren Ende des Feuerzeugs unter der Kammer 6 für den Brennstoff eine kleine weitere Kammer 30 belegen, die ein Kondom 11 aufnehmen kann, das jedoch um 90° gedreht gegenüber der Ausführungsform von Fig. 2 eingebracht ist. Die nach unten gerichtete freie Öffnung der Kammer 30 wird durch einen federnden Deckel 31 verschlossen, der auch durch einen Schraubverschluß ersetzt sein kann.

## Patentansprüche

1. Wegwerffeuerzeug mit Kondom, bei dem das Feuerzeug (1) angrenzend an den Brennstofftank (2) eine Kammer (10) zur Aufnahme des Kondoms (11) aufweist, die den unteren Bereich des Feuerzeugs ausfüllt und eine wiederverschließbare Entnahmeöffnung (13) aufweist, wobei das Kondom von einer Schutzhülle umschlossen ist, die eine Lasche (12) enthält, welche ein zerstörungsfreies Herausziehen des Kondoms ermöglicht.

2. Wegwerffeuerzeug nach Anspruch 1, dadurch gekennzeichnet, daß an der Unterseite des Feuerzeugs (1) eine Klappe (15) angebracht ist, welche zumindest einen Teil des Bodens des Feuerzeugs (1) bildet.

3. Wegwerffeuerzeug nach Anspruch 2, dadurch gekennzeichnet, daß die Klappe eine lösbare Arretierung (16) aufweist.

4. Wegwerffeuerzeug nach Anspruch 1, dadurch gekennzeichnet, daß die Kammer (20) an einer Seitenfläche und/oder an der Unterseite des Feuerzeugs (1) offen und durch eine über den Körper des Feuerzeugs (1) aufschiebbare Hülse (24) abdeckbar ist.

5. Wegwerffeuerzeug mit Kondom, bei dem das Feuerzeug (1) angrenzend an den Brennstofftank (2) eine Kammer (10) zur Aufnahme des Kondoms aufweist, wobei die Kammer hermetisch abgeschlossen ist und wobei ein Zugang zum Kammerinneren durch Zerstörung der Kammerwandung (17) längs einer Sollbruchstelle (18) vorgesehen ist.

6. Wegwerffeuerzeug nach Anspruch 5, gekennzeichnet durch eine Lasche (19) zum Aufreißen der Kammerwandung längs der Sollbruchstelle (18).

7. Wegwerffeuerzeug nach Anspruch 5 oder 6, dadurch gekennzeichnet, daß der Zugang zu der Kammer (10) im Seitenbereich des Feuerzeugs (1) vorgesehen ist.

8. Wegwerffeuerzeug nach einem der Ansprüche 1 bis 6, dadurch gekennzeichnet, daß der Zugang (13;23) zu der Kammer (10;20) an der Unterseite des Feuerzeugs (1) vorgesehen ist.

9. Wegwerffeuerzeug nach einem der vorstehenden Ansprüche, dadurch gekennzeichnet, daß der Brennstofftank (6) und die Wandung der Kammer aus Kunststoff bestehen.

10. Wegwerffeuerzeug nach Anspruch 9, dadurch gekennzeichnet, daß zumindest die Brennstoffkammer und/oder die Wandungen der Kammer (10;20;30) aus einem einstückigen Kunststoffteil bestehen.

11. Wegwerffeuerzeug nach Anspruch 9 oder 10, dadurch gekennzeichnet, daß die Außenwand des Kunststoffteils zumindest im Bereich der Kammer (10;20) undurchsichtig ist.

12. Wegwerffeuerzeug nach Anspruch 9 oder 10, dadurch gekennzeichnet, daß zumindest die Außenwand der Kammer (10;20;30) aus einem durchsichtigen Kunststoff gefertigt ist.

13. Wegwerffeuerzeug nach einem der Ansprüche 1 bis 4, dadurch gekennzeichnet, daß das Kondom (11) in einer farbigen oder gemusterten Schutzhülle aufgenommen und mit dieser in die Kammer (10;20;30) eingebracht ist, welche eine durchsichtige oder durchscheinende Wandung aufweist.

## Claims

1. A disposable lighter with condom, wherein the lighter (1) comprises, adjacent to the fuel reservoir (2), a compartment (10) for receiving the condom (11), said compartment occupying the lower portion of the lighter and being provided with a reclosable withdrawal opening (13), the condom being enclosed by a protective cover equipped with a tab (12) which allows the condom to be withdrawn without being damaged.

2. The disposable lighter of claim 1, characterized in that a flap (15) is provided on the underside of the lighter (1) and forms at least part of the bottom of the lighter (1).

3. The disposable lighter of claim 2, characterized in that the flap is provided with a releasable locking mechanism (16).

4. The disposable lighter of claim 1, characterized in that the compartment (20) is open at a lateral wall and/or the underside of the lighter (1) and coverable by a sleeve (24) which can be shifted over the body of the lighter (1).

5. A disposable lighter with condom, wherein the lighter (1) comprises, adjacent to the fuel reservoir (2), a compartment (10) for receiving the condom, said compartment being hermetically sealed and an access to the interior of said compartment being provided in that the compartment wall (17) is destroyed along a predetermined breaking line (18).

6. The disposable lighter of claim 5, characterized by a tab (19) for tearing the compartment wall open along the predetermined breaking line (18).

7. The disposable lighter of claim 5 or 6, characterized in that the access to the compartment (10) is provided in the side portion of the lighter (1).

8. The disposable lighter of one of claims 1 to 6, characterized in that the access (13; 23) to the compartment (10; 20) is provided at the underside of the lighter (1).

9. The disposable lighter of one of the preceding claims, characterized in that the fuel reservoir (6) and the wall of the compartment are made of plastic material.

10. The disposable lighter of claim 9, characterized in that at least the fuel reservoir and/or the walls of the compartment (10; 20; 30) are made from a unitary plastic part.

11. The disposable lighter of claim 9 or 10, characterized in that the outer wall of the plastic part is nontransparent at least in the area of the compartment (10; 20).

12. The disposable lighter of claim 9 or 10, characterized in that at least the outer wall of the compartment (10; 20; 30) is made from a transparent plastic material.

13. The disposable lighter of one of claims 1 to 4, characterized in that the condom (11) is received in a coloured or patterned protective cover and accommodated with the latter in the compartment (10; 20; 30) which is formed with a transparent or translucent wall.

**Revendications**

1. Briquet jetable avec condom dans lequel le briquet (1) comporte, contre le réservoir d'essence (2), un compartiment (10) destiné à loger le condom (11), qui remplit la région inférieure du briquet et présente une ouverture de prélèvement (13) pouvant être refermée, le condom étant entouré d'une enveloppe de protection qui présente une patte (12) permettant d'extraire le condom sans le détruire.

2. Briquet jetable selon la revendication 1, caractérisé en ce que la face inférieure du briquet (1) est munie d'un volet (15) qui forme au moins une partie du fond du briquet (1).

3. Briquet jetable selon la revendication 2, caractérisé en ce que le volet comporte un dispositif de verrouillage (16) amovible.

4. Briquet jetable selon la revendication 1, caractérisé en ce que le compartiment (20) est ouvert sur la face latérale et/ou sur la face inférieure du briquet (1) et peut être recouvert par une douille (24) à enfiler sur le corps du briquet (1).

5. Briquet jetable avec condom dans lequel le briquet (1) comporte, contre le réservoir d'essence (2), un compartiment (10) destiné à loger le condom, le compartiment étant hermétiquement fermé et un accès à l'intérieur du compartiment étant prévu, par destruction de la paroi (17) du compartiment le long d'une ligne de rupture (18).

6. Briquet jetable selon la revendication 5, caractérisé en ce qu'il est prévu une patte (19) pour ouvrir la paroi du compartiment le long de la ligne de rupture (18).

7. Briquet jetable selon la revendication 5 ou 6, caractérisé en ce que l'accès au compartiment (10) est prévu dans la région latérale du briquet (1).

8. Briquet jetable selon l'une des revendications 1 à 6, caractérisé en ce que l'accès (13 ; 23) au compartiment (10 ; 20) est prévu sur la face inférieure du briquet (1).

9. Briquet jetable selon l'une des revendications précédentes, caractérisé en ce que le réservoir d'essence (6) et la paroi du compartiment sont en matière plastique.

10. Briquet jetable selon la revendication 9, caractérisé en ce que le réservoir d'essence et/ou les parois du compartiment (10 ; 20 ; 30) au moins sont constitués d'un élément en matière plastique d'une seule pièce.

11. Briquet jetable selon la revendication 9 ou 10, caractérisé en ce que la paroi extérieure de l'élément en matière plastique est opaque au moins dans la région du compartiment 10 ; 20).

12. Briquet jetable selon la revendication 9 ou 10, caractérisé en ce qu'au moins la paroi extérieure du compartiment (10 ; 20 ; 30) est réalisée dans une matière plastique transparente.

13. Briquet jetable selon l'une des revendications 1 à 4, caractérisé en ce que le condom (11) est logé dans une enveloppe de protection colorée ou à motifs et est placé avec celle-ci dans le compartiment (10 ; 20 ; 30) qui présente une paroi transparente ou translucide.

**Fig. 1a**

**Fig. 1b**

*Fig. 2*

*Fig. 3*